(19) Europäisches Patentamt
European Patent Office
Office européen des brevets

(11) **EP 4 226 857 A1**

(12) **EUROPEAN PATENT APPLICATION**

(43) Date of publication:
**16.08.2023 Bulletin 2023/33**

(21) Application number: **22155813.3**

(22) Date of filing: **09.02.2022**

(51) International Patent Classification (IPC):
**A61B 5/026** (2006.01)    **A61B 90/20** (2016.01)

(52) Cooperative Patent Classification (CPC):
**A61B 5/026; A61B 90/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Leica Instruments (Singapore) Pte Ltd
Singapore 608924 (SG)**

(72) Inventor: **Díez-Ochoa, Miguel
9435 Heerbrugg (CH)**

(74) Representative: **DehnsGermany Partnerschaft
von Patentanwälten
Theresienstraße 6-8
80333 München (DE)**

(54) **IMAGE PROCESSING SYSTEM AND METHOD FOR ANALYSING IMAGES, CONTROLLER AND SURGICAL MICROSCOPE SYSTEM**

(57)    The invention relates to an image processing system (150) for analysing images of a tissue for blood flow, configured to: determine, in a time series (120) of images (122a, 122b, 122c, 124a, 124b, 124c) of a tissue, a region in each of said images (122a, 122b, 122c, 124a, 124b, 124c), in which a colour or intensity varies over time; generate, based on said region, data (128), which comprises information characteristic for blood flowing through a blood vessel in said tissue; and provide said data (128). The invention also relates to a corresponding controller, surgical microscope system and method.

**Fig. 1**

EP 4 226 857 A1

**Description**

**Technical Field**

The present invention essentially relates to an image processing system and a method for analysing images of a tissue for blood flow, to a controller for a surgical microscope and to a surgical microscope system.

**Background**

In surgical microscopy, for example, blood flow in tissue often has to be detected such that a surgeon is aware of where blood vessels are and where not. To detect how blood flows through vessels, a fluorescence substance, e.g., indocyanine green ("ICG") can be injected into the patient undergoing the surgery. ICG, when illuminated with light in the red area, emits light in the infra-red. This light can be captured by near infrared cameras and displayed, so the surgeon can have a better understanding of the situation. A problem of this method is the short lifetime of ICG, which is about three minutes. Another problem is related to the toxicity of the ICG. As this drug is toxic for the human body, it cannot be injected continuously. So, the surgeon cannot have this information during the whole surgery.

**Summary**

In view of the situation described above, there is a need for improvement in detecting blood flow in tissue. According to embodiments of the invention, an image processing system and a method for analysing images of a tissue for blood flow, a controller for a surgical microscope and a surgical microscope system with the features of the independent claims are proposed. Advantageous further developments form the subject matter of the dependent claims and of the subsequent description.

An embodiment of the invention relates to an image processing system for analysing images of a tissue for blood flow. The image processing system can comprise one or more processors and one or more storage devices. The image processing system is configured to determine, in a time series of images of a tissue (i.e., with the same tissue in each image; said tissue should comprise a blood vessel), a region in each of said images, in which a colour or intensity varies over time, i.e., varies from image to image. These images can be acquired with a single spectrum or with multiple spectra. Preferably, visible light and/or infra-red light are used; this enhances the data basis. The image processing system is further configured to generate, based on said region, data, which comprises information that is characteristic for blood flowing through a blood vessel in said tissue, and to provide said data. In an embodiment of the invention, the image processing system is configured to receive said time series of images from a surgical microscope or an image detector of a surgical microscope.

Images of blood vessels can contain information about how the blood is flowing through the vessels, which is, however, not perceivable to the human eye. It has now turned out that this information can be provided, e.g., to the surgeon, by means of image processing. When blood is pumped through vessels, it produces small variations in the colour of the vessel. These variations might not be perceptible to the human eye, but they can be captured with cameras and, e.g., amplified to make them visible. This allows the surgeon or any other person to make use of said information that is characteristic for blood flowing through a blood vessel, e.g., during a surgery. It is noted that within each of such images, more than one of said regions might be present and that each of such images might show more than one vessel. The described method can be applied to each of them. Ideally, such region with colour or intensity varying over time corresponds to a blood vessel; however, in practice not all parts of a blood vessel may exhibit sufficient colour or intensity variation and/or other parts than a blood vessel might show high colour or intensity variation. However, it has turned out that the overlap is sufficient to identify blood vessels.

According to an embodiment of the invention, said data, which comprises information, which is characteristic for blood flowing through a blood vessel in said tissue, comprises: a blood flow image, which indicates blood flowing through a blood vessel in said tissue. In this way, the surgeon, for example, can see where blood is flowing or where blood vessels are, e.g., in real-time during a surgery. No toxic drugs have to be provided to the patient; in particular, there has to be no interaction with the patient at all, only images have to be acquired.

Such blood flow image as such, preferably, is an image separate from the (original) images of said tissue. This allows, in an embodiment of the invention, to provide said blood flow image being overlaid onto one of said images of said time series. In this way, the surgeon, for example, can have a direct view of where blood vessels are, in the (original) image or (original) view. According to a further embodiment of the invention, said blood flow image can be provided in addition to one of said images of said time series; for example, said blood flow image might be displayed (on a display) next to such image or with picture-in-picture view. Also, a separate display might be used to present said blood flow image. This allows a different view to the blood flow.

According to a further embodiment of the invention, the image processing system is further configured to determine a magnitude of a variation of said colour or intensity within said region; and to generate said blood flow image based one of said images of said time series by amplifying said magnitude in said region. Said magnitude of a variation can, e.g., be a maximum variation with respect to a mean value. This allows easy and efficient visualization of the required intensity or colour variation. Preferably, the image processing system is further configured to determine whether a colour or intensity varies over time, within said region, with a frequency within a pre-determined frequency range. Such pre-determined frequency range is, in an embodiment of the invention, at least one of: determined based on a person's heart-rate, from which person the images are obtained, e.g., via (external) heart-rate monitoring; and set based on a standard or typical range of human heart-rates (e.g., the range might be set to between 40 Hz and 180 Hz). This allows very efficient selection of the frequency of interest and, in addition, neglecting frequencies, which are not of interest, easily.

According to a further embodiment of the invention, the image processing system is configured to determine said region having a colour or intensity varying over time by: determining an intensity of one or more or all pixels of each of said images as a function of time; and by: determining time dependent parts of said function corresponding to said pre-determined frequency range. This allows easy and efficient image processing. For example, a Fourier Transformation, a Fast Fourier Transformation or a similar method might be used here. The analysis of the pixels in the image results in a 'delta' value (said magnitude mentioned above) for each pixel; the set of all contiguous pixels with a 'delta' good enough (e.g., more than a pre-defined value) corresponds to the region.

According to a further embodiment of the invention, the image processing system is further configured to remove from said images, before determining said region, at least part of noise. Such noise to be removed can have frequencies outside said pre-determined frequency range. Such noise might be removed by using, e.g., a (noise) filter. Also, noise inside that pre-determined frequency range might be present, that can be removed. This improves determining the required frequency of the blood flow, which corresponds to the heart-rate.

According to a further embodiment of the invention, the image processing system is further configured to correct said time series of images for motion by tracking said blood vessel through the images, before determining whether a colour or intensity varies over time. In this way, optical flow can be considered. Optical flow means that the position of specific blood vessels and, thus, said region or image, might slightly change over time due to slight movement of the patient and/or the surgical microscope (which includes the image sensor). Images usually are not static, thus, tracking the position of each pixel of interest along time is of advantage. This tracking allows to measure the changes of intensity on the same portion of the object. Otherwise, intensities of different portions of the object might be compared and the measure will not be valid or, at least, of less quality.

According to a further embodiment of the invention, the image processing system is further configured to: receive said time series of images one image after the one; and to determine, whether a colour or intensity varies over time, based on a pre-defined number of latest images. This can correspond to a FIFO ("First In First Out"), update buffer with the latest images and allows very efficient image processing.

According to a further embodiment of the invention, said data, which comprises information, which is characteristic for blood flowing through a blood vessel in said tissue, comprises: a frequency with which said colour or intensity varies over time. Said data is then provided as a heart-rate. As the frequency, which with the blood is pumped corresponds to the (current) heart-rate of the patient, this allows a very easy and efficient way of providing the current heart-rate of the patient, e.g., during a surgery. Such heart-rate might be display on a display next to the blood flow image, for example.

A further embodiment of the invention relates to a controller for a surgical microscope, which controller is configured as the image processing system of any one of the embodiments mentions, or, which comprises the image processing system of any one of the above-mentioned embodiments.

A further embodiment of the invention relates to a surgical microscope system, comprising a surgical microscope, an image detector and the controller according to any of the above-mentioned embodiments. Depending on the number of spectra to be acquired, more than one image detector might be required.

According to a further embodiment of the invention, the surgical microscope system further comprises a display configured for displaying said data, which comprises information characteristic for blood flowing through a blood vessel in said tissue, e.g., the blood flow image and/or the heart-rate.

A further embodiment of the invention relates to a method for analysing images of a tissue for blood flow. The method comprises: receiving a time series of images of a tissue; determining a region in each of said images, in which a colour or intensity varies over time; generating, based on said region, data, which comprises information characteristic for blood flowing through a blood vessel in said tissue; and providing said data. Preferably, said data comprises: a blood flow image (126), which indicates blood flowing through a blood vessel in said tissue. Also, said data can comprise a heart-rate. According to an embodiment of the invention, the method further comprises: acquiring the time series of images, before receiving, by means of an image detector of a surgical microscope.

With respect to advantages and further embodiments of the controller, the surgical microscope system and the method, it is referred to the remarks of the image processing system, which apply here correspondingly.

A further embodiment of the invention relates to a computer program with a program code for performing the method of above, when the computer program is run on a processor.

Further advantages and embodiments of the invention will become apparent from the description and the appended figures.

It should be noted that the previously mentioned features and the features to be further described in the following are usable not only in the respectively indicated combination, but also in further combinations or taken alone, without departing from the scope of the present invention.

**Short description of the Figures**

Fig. 1    schematically shows an image processing system according to an embodiment of the invention;

Fig. 2    schematically shows an image processing system according to a further embodiment of the invention;

Fig. 3    schematically shows a time series of images to be analyzed;

Fig. 4    schematically shows how to analyze the images of Fig. 3; and

Fig. 5    schematically shows a method according to an embodiment of the invention.

**Detailed Description**

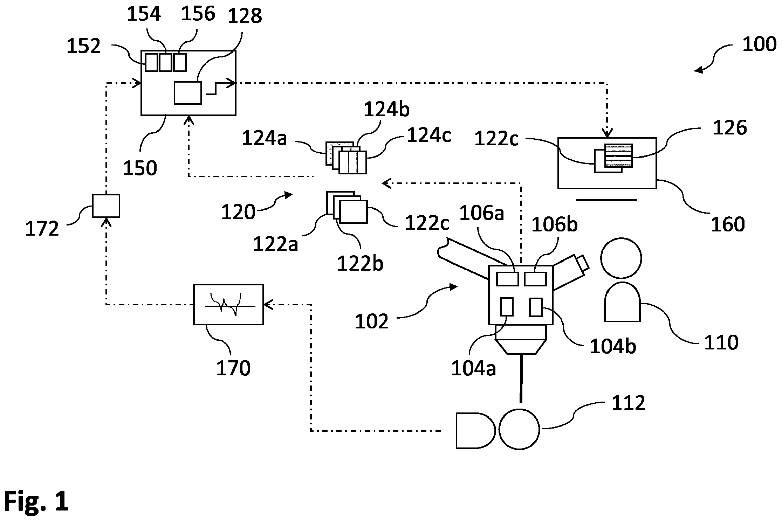

Fig. 1 schematically illustrates an image processing system 150 according to an embodiment of the invention. The image processing system 150 comprises, in an embodiment of the invention, one or more processors (CPUs) 152, one or more storage devices 154, and one or more graphic processors (GPUs) 156.

Further, Fig. 1 schematically illustrates a surgical microscope system 100 according to an embodiment of the invention. Said surgical microscope system 100 comprises a surgical microscope 102, a display 160 and said image processing system 150, which can be, in an embodiment of the invention, a controller for said surgical microscope 102.

Said surgical microscope 102 can comprise an illumination optics 104a for visible light and an illumination optics 104b for infra-red light for illuminating tissue of a patient 112. An image detector 106a, e.g., a sensor or camera, acquires visible light, emanating from the illuminated tissue. Image detector 106b, e.g., a sensor or camera, acquires infra-red light emanating from the illuminated tissue. Note that other and/or different spectra for illuminating and/or detecting can be used. Depending on the specific situation, necessary illumination optics and appropriate images detector (one or more) shall be used. During a surgery, a surgeon 110 (user) can use said surgical microscope 102 in order to view the surgical site, e.g., said patient 112 or tissue like a patient's brain.

Image processing system 150 is configured to determine, in a time series 120 of images 122a, 122b, 122c, 124a, 124b, 124c of a tissue, a region in each of said images, in which a colour or intensity varies over time. An example of such image(s) and a preferred way of how to determine said colour or intensity variation will be described later. This might include considering the current heart-rate 172 of patient 112, which can be measured by means of an (external) heart-rate monitor 170, for example.

In an embodiment of the invention, the images 122a, 122b, 122c, 124a, 124b, 124c are received from said surgical microscope 102 or one or more of said image detectors 106a and 106b of said surgical microscope 102, e.g., one image after the other. Also, corresponding visible light and infra-red light images might be received simultaneously. Said images can comprise, for example, visible light images 122a, 122b, 122c and infra-red light images 124a, 124b, 124c. Also, other and/or different spectra might be included in such images. Such images with different spectra can be handled in different (e.g., two different) time series. Also, a single time series with combined images might be used; in such case, multi-channel images can be used or created. For example, visible light image 122a and infra-red light image 124a might be combined to a four-channel (RGB and IR) image.

Image processing system 150 is further configured to generate, based on said region, data 128, which comprises information, which is characteristic for blood flowing through a blood vessel in said tissue. According to an embodiment of the invention, these data 128 comprise a blood flow image 126, which indicates blood flowing through a blood vessel in said tissue.

Image processing system 150 is further configured to provide said data 128, preferably, said blood flow image 126. For example, said blood flow image 126 can be provided overlaid onto one of said images, e.g., image 122c, of said time series 120 and, e.g., be displayed or presented on said display 160. Alternatively, or in addition, said blood flow image 126 can also be provided and displayed in addition to said image 122c, for example, next to it or by way of picture-in-picture.

In this way, the surgeon can be provided with information about blood vessels and their location within the tissue, e.g., the brain of the patient 112, which is undergoing a surgery. Note that such determining of a blood flow image, which indicates blood flowing through a blood vessel in a tissue, from a time series of images, can - in general - be performed independently from such surgery, e.g., later for further analysis. However, real-time use brings specific benefit for the surgeon during the surgery.

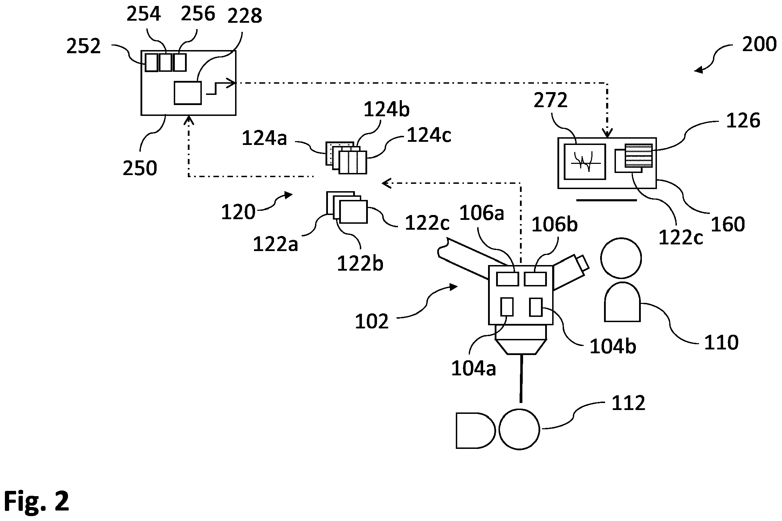

Fig. 2 schematically illustrates an image processing system 250 according to a further embodiment of the invention. The image processing system 250 comprises, in an embodiment of the invention, one or more processors (CPUs) 252, one or more storage devices 254, and one or more graphic processors (GPUs) 256.

Further, Fig. 2 schematically illustrates a surgical microscope system 200 according to an embodiment of the invention. Said surgical microscope system 200 comprises a surgical microscope 102, a display 160 and said image processing system 250, which can be, in an embodiment of the invention, a controller for said surgical microscope 102. Note that the surgical microscope system 200 can correspond to surgical microscope system 100 of Fig. 1, using, however, image processing system 250 instead of image processing system 150.

Image processing system 250 is configured to determine, in said time series 120 of images 122a, 122b, 122c, 124a, 124b, 124c) of a tissue, a region in each of said images, in which a colour or intensity varies over time; and to generate, based on said region, data 228, which comprises information characteristic for blood flowing through a blood vessel in said tissue; and to provide said data. This is similar to the situation in Fig. 1 and image processing system 150.

In addition to data 128 of Fig. 1, data 228 further comprises a frequency with which said colour or intensity varies over time, and is, then, provided as (current or real-time) heart-rate 272 of said patient 112. Said heart-rate is, for example, displayed on display 160 such that surgeon 110 can consider the current heart rate. Heart-rate monitor 170 of Fig. 1 is, thus, not necessary.

It is noted that determining the frequency with which said colour or intensity varies over time, and, thus, heart-rate 272 of said patient 112 can be performed independently from and without determining the blood flow image, in case only a current heart-rate is required. In this case, image processing system 250 can be considered as a heart-rate monitoring system or as a part thereof.

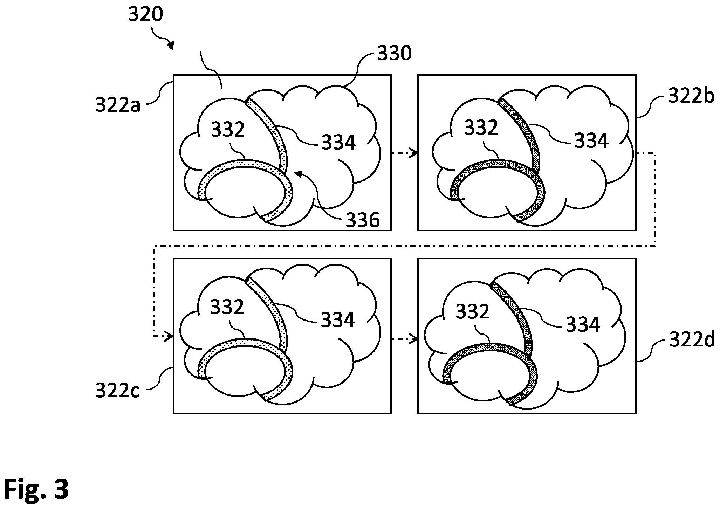

Fig. 3 schematically illustrates a time series 320 of images 322a, 322b, 322c, 322d to be analyzed; the sequence of the images in which they are acquired and received is indicated with dashed lined arrows from image to image. Time series 320 and its images can correspond to time series 120 of Fig. 1 with its images 122a,122b, 122c(by means of example visual light images only, with here four instead of three images). Such images 322a, 322b, 322c, 322d can be acquired in real-time with a certain imaging frequency, for example 120 Hz, and received at the image processing system. Thus, in practice, many more images will be acquired and analyzed; the four images shown here are just for explanation. Also note that typically only a certain number of images are used for analyzing, for example, the latest 120 images or the like (see the FIFO buffer mentioned above).

Image 322a shows, by means of example, a tissue 330, e.g., a brain or part thereof, and blood vessels 332, 334. Images 322b, 322c and 322d also show said tissue 330 and the blood vessels 332, 336. There are different aspects shown in the time series 320. An aspect is that the position of tissue 330 and the blood vessels 332, 334 varies from image to image; for example, tissue 330 and blood vessels 332, 334 are more at the right margin in image 322b, compared to image 322a. Such movement is known as optical flow and results, for example, from slight movement of the surgical microscope and its imaging detector(s) during acquiring these images and/or a slight movement of the patient and, thus, the tissue during acquiring these images. Thus, the position of each pixel of interest can be tracked along time. This tracking allows us to measure the changes of intensity on the same portion of the object as will be explained below. Thus, said time series 320 of images is corrected for motion by tracking said blood vessel(s) 322, 334 through the images 332a, 322b, 322c, 322d, before determining whether a colour or intensity varies over time. This can be done by correcting each newly received image with respect to the ones received before (if using the FIFO buffer, for example). Such correction can comprise, for example, a lateral movement displacement of the content of the image within the image's margins.

Another aspect shown here is that the colour or intensity of the blood vessels as shown in the respective image vary over time and, thus, from image to image. This is indicated here with a lighter shading of blood vessels 332, 334 in images 322a and 322c and a darker shading in images 322b and 322d. The reason for such colour or intensity variation of the blood vessels is the blood being pumped through the blood vessels by the patient's heart; the increased blood pressure in the vessels after a pumping action results in such slight colour change. Thus, the colour or intensity variation has a frequency that (at least basically) corresponds to the patient's (current) heart-rate.

It is noted that such colour or intensity variation in these images is typically not perceivable by human eyes. The variation shown here is just for illustration purposes. Thus, a surgeon looking at the tissue in reality (or via the surgical microscope) can, for example, not see whether blood is flowing through the blood vessels and, further, even might not see whether a vessel or certain part of said tissue is a blood vessel at all.

However, such colour or intensity variation can be identified by image analysis and processing; the blood vessels with colour or intensity variation appears as a region with colour or intensity variation during image analysis; note that the processing system does not know that there is a blood vessel, but can determine a region with colour or intensity variation. Thus, a region 336 is determined in each of said images 322a, 322b, 322c, 322d, in which region a colour or intensity varies over time; as mentioned before, such region ideally correspond to blood vessels. Correcting for motion, as mentioned above, allows comparing each individual pixel within said region 336 for colour or intensity variations from image to image. Note that such region like region 336 can differ in size. Also, two or more regions (which might then also be smaller) may be identified in which colour or intensity variations occur; this can be the case, for example, if several blood vessels not connected to each other are present or if part of a blood vessel does not exhibit sufficient variation in colour or intensity.

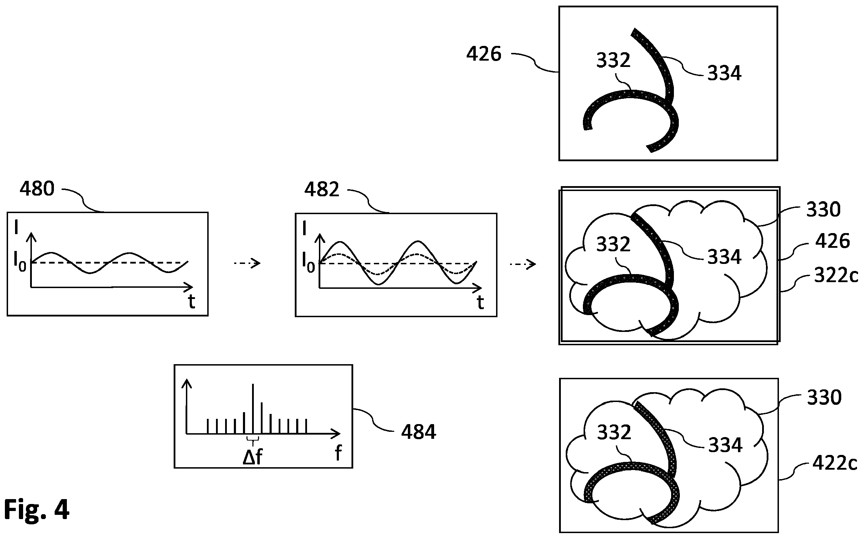

Fig. 4 schematically illustrates how to analyze the images of Fig. 3. For each pixel of said images 322a, 322b, 322c, 322d, a variation in intensity or colour can be determined. Such variation will be found (typically) only for pixels which show a blood vessel with blood flowing through it. These pixels exhibiting a variation correspond to the region mentioned. Such variation in intensity $I$ over time t is shown, for one such pixel, by means of example, in diagram 480. Intensity $I$ can be represented as a function of position x, y (defining the position of the pixel, spatial coordinates) and time t with the following equation:

$$I(x, y, t) = f(x, y) + \delta(x, y, t) + \varepsilon(x, y, t)$$

Assuming a static object, the intensity of a pixel is given by a function f dependent of the spatial coordinates (x, y), another function $\delta$ dependent on the spatial and temporal (t, time) coordinates and some noise $\varepsilon$. For an individual pixel (x and y fixed), the part $f(x, y)$ is static and can be considered a mean or fixed value like $I_0$ shown in diagram 480. After having removed noise, the curve shown with a full line in diagram 480 corresponds to function $\delta = \delta(t)$.

A goal is to extract $\delta$ and, preferably, amplify it until it is perceivable to the human eye. This $\delta$ has the characteristic of following a pattern, and this pattern should be the same for every pixel of interest (pixel showing a blood vessel with blood flowing through) as this pattern is driven by the heart pumping blood. Once the differences related to the blood flow have been extracted and, preferably, amplified by a factor $\alpha$, the output image can have the following equation:

$$\bar{I}(x, y, t) = f(x, y) + \alpha * \delta(x, y, t) + \varepsilon(x, y, t)$$

Such amplified intensity variation, $\alpha * \delta(x, y, t)$, is shown in diagram 482 with the curve shown with a full line (the curve in dotted line corresponds to $\delta(t)$, like in diagram 480). Such amplified intensity or colour variation is then perceivable to the human eye, because the human eye is able to differentiate between the lowest and highest intensity.

According to an embodiment of the invention, determining whether a colour or intensity varies over time, within said region, occurs with a certain frequency is performed within a pre-determined frequency range $\Delta f$ as illustrated in diagram 484. Such frequency range $\Delta f$ can be determined based on the current heart-rate of the patient, as illustrated in Fig. 1 (e.g., the current heart-rate +/-10%) or it can be set to a typical range of heart-rates of patients. This allows finding the required frequency, for which the variation is to amplify, much easier.

A blood flow image 426 can be created from such region with intensity or colour variation, i.e., all pixels exhibiting a $\delta(t) \neq 0$; such blood flow image 426, for example, only shows the blood vessel and is illustrated on the top right of Fig. 4. The content (vessels) shown in such blood flow image can, for example, have the colour or intensity, which corresponds to the amplified intensity or colour variation. Also, the content (vessels) shown in such blood flow image can, for example, have any different colour like green in order to more clearly indicate the locations of the blood vessels; such blood flow image can be considered a pseudo-colour image.

Further, such flow image 426 can be overlaid onto one of said images of said time series, for example image 322c as illustrated on the middle right of Fig. 4 (note that the slight mismatch of blood flow image 426 and image 322c is just to illustrate the overlay; in practice, both images should be exactly matched).

Further, the blood flow image, in an embodiment of the invention, can comprise the original image like image 322c with the blood vessels changed (amplified) in colour. In other words, in original image 322c, the pixels exhibiting said intensity or colour variation might be amplified (resulting in a colour change) according to the formula above. The result is image 422c as illustrated on the bottom right of Fig. 4, corresponding to image 322c with the region (blood vessels 332, 334) being amplified. Image 422c is, thus, also a blood flow image.

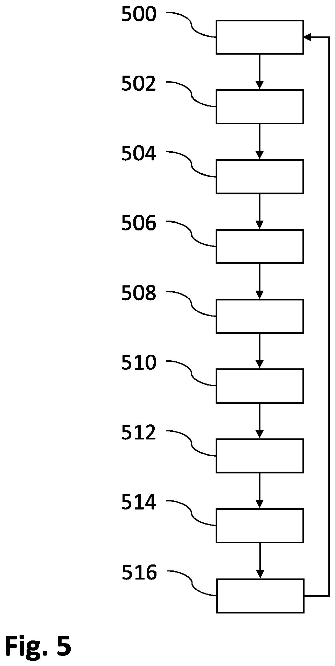

Fig. 5 schematically illustrates a method according to an embodiment of the invention by means of a flow diagram. In step 500, a new image of a tissue of, e.g., a patient, is acquired and received, e.g., in an image processing system. In step 502, noise is reduced in said newly acquired and received image. This might comprise applying a noise filter in order to reduce noise, which has frequencies that are not of interest In step 504, said image is corrected for motion (optical flow) by tracking a blood vessel in said image through other images acquired and received before. It is noted that this step is not performed for the very first image of a time series that has been received.

In step 506, a buffer, in which subsequent images that have been received, are stored, is updated. Such buffer, for example provided by the image processing system, can store a certain number of images. Each newly acquired, received image is stored therein. If the buffer has reached its maximum number of images, the oldest image stored therein is removed in order to store the latest image (FIFO). In this way, a time series of images is received, see step 508, e.g., at the image processing system.

In step 510, a region in each of said images is determined, in which a colour or intensity varies over time; in other words, a temporal analysis is performed on the time series of images or the region determined therein, respectively. In step 512, colour or intensity variations (or its magnitudes) are amplified. Such temporal analysis and magnification have been described in detail with respect to Figs. 3 and 4.

In step 514, based on said region, data, which comprises information characteristic for blood flowing through a blood vessel in said tissue, is generated. Preferably, said data comprises a blood flow image indicates blood flowing through a blood vessel in said tissue. In step 516, said data or blood flow image is provided and, for example, displayed on a display.

In this way, the latest image that has been acquired and received is used to generate the blood flow image. Afterwards, the method can again start with step 500 and acquiring and receiving a new image.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1 to 5. Alternatively, a microscope may be part of or connected to a system as described in connection with one or more of the Figs. 1 to 5. Fig. 1 shows a schematic illustration of a surgical microscope system 100 configured to perform a method described herein. The surgical microscope system 100 comprises a surgical microscope 102 and an image processing or computer system 150. The microscope 102 is configured to take images and is connected to the computer system 150. The computer system 150 is configured to execute at least a part of a method described herein. The computer system 150 may be configured to execute a machine learning algorithm. The computer system 150 and microscope 102 may be separate entities but can also be integrated together in one common housing. The computer system 150 may be part of a central processing system of the microscope 102 and/or the computer system 150 may be part of a subcomponent of the microscope 102, such as a sensor, an actor, a camera or an illumination unit, etc. of the microscope 102.

The computer system 150 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 150 may comprise any circuit or combination of circuits. In one embodiment, the computer system 150 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 150 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 150 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 150 may also include a display device, one or more speakers, and a keyboard and/or controller, which can include a mouse, trackball, touch screen, voice-recognition device, or any other device that permits a system user to input information into and receive information from the computer system 150.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

**List of Reference Signs**

| | |
|---|---|
| 100,200 | surgical microscope system |
| 102 | surgical microscope |
| 104a, 104b | illumination optics |
| 106a, 106b | imaging detector |
| 110 | user |
| 112 | patient |
| 120, 320 | time series of images |
| 122a, 122b, 122c, 124a, 124b, 124c, 322a, 322b, 322c, 322d | Images |
| 126,426,422c | blood flow image |
| 128,228 | data |
| 150, 250 | image processing system |
| 152,252 | processor |
| 154,254 | storage device |
| 156, 256 | graphic processor |
| 160 | display |
| 170 | heart-rate monitor |
| 172,272 | heart-rate |
| 330 | tissue |
| 332,334 | blood vessel |

EP 4 226 857 A1

336 region

480, 482, 484 diagrams

500-516 method steps

**Claims**

1. An image processing system (150, 250) for analysing images of a tissue for blood flow, configured to:

   determine, in a time series (120, 320) of images (122a, 122b, 122c, 124a, 124b, 124c, 322a, 322b, 322c, 322d) of a tissue, a region (336) in each of said images (122a, 122b, 122c, 124a, 124b, 124c, 322a, 322b, 322c, 322d), in which a colour or intensity varies over time;
   generate, based on said region (336), data (128, 228), which comprises information characteristic for blood flowing through a blood vessel in said tissue; and
   provide said data (128, 228).

2. The image processing system (150, 250) of claim 1, wherein said data (128, 228), which comprises information, which is characteristic for blood flowing through a blood vessel (332, 334) in said tissue (330), comprises: a blood flow image (126, 426, 422c), which indicates blood flowing through a blood vessel in said tissue.

3. The image processing system (150, 250) of claim 2, further configured to:
   provide said blood flow image (126, 426) being overlaid onto one of said images (122c, 322c) of said time series (120, 320).

4. The image processing system (150) of claim 2 or 3, further configured to:
   provide said blood flow image (126, 426, 422c) in addition to or comprising one of said images (122c, 322c) of said time series (120, 320).

5. The image processing system (15, 2500) of any one of claims 2 to 4, further configured to:

   determine a magnitude of a variation of said colour or intensity within said region (336); and
   generate said blood flow image (126, 426) based on one of said images of said time series by amplifying said magnitude in said region (336).

6. The image processing system (150, 250) of any one of claims 2 to 5, further configured to determine whether a colour or intensity varies over time, within said region (336), with a frequency within a pre-determined frequency range ($\Delta f$).

7. The image processing system (150, 250) of claim 6, wherein said pre-determined frequency range ($\Delta f$) is at least one of:

   - determined based on a person's heart-rate (172), from which person (112) the images are obtained, and
   - set based on a standard or typical range of human heart-rates.

8. The image processing system (150, 250) of claim 6 or 7, configured to determine said region (336) having a colour or intensity varying over time by:

   determining an intensity (I) of one or more or all pixels of each of said images (122a, 122b, 122c, 124a, 124b, 124c, 322a, 322b, 322c, 322d) as a function of time; and
   determining time dependent parts of said function corresponding to said pre-determined frequency range ($\Delta f$).

9. The image processing system (150, 250) of any one of claims 5 to 7, further configured to remove from said images, before determining said region, at least part of noise.

10. The image processing system (150, 250) of any one of the preceding claims, further configured to correct said time series (120, 320) of images (122a, 122b, 122c, 124a, 124b, 124c, 322a, 322b, 322c, 322d) for motion by tracking said blood vessel (332, 334) through the images (122a, 122b, 122c, 124a, 124b, 124c, 322a, 322b, 322c, 322d), before determining whether a colour or intensity varies over time.

11. The image processing system (150, 250) of any one of the preceding claims, wherein said images (120) are obtained using illumination of said blood vessel by means of at least one of: visible light, infra-red light.

12. The image processing system (150, 250) of any one of the preceding claims, further configured to:

   receive said time series (120, 320) of images one image after the one; and
   determine, whether a colour or intensity varies over time, based on a pre-defined number of latest images.

13. The image processing system (250) of any one of the preceding claims, wherein said data (228), which comprises information, which is characteristic for blood flowing through a blood vessel in said tissue, comprises: a frequency with which said colour or intensity varies over time; and
   wherein said data is provided as a heart-rate (272).

14. The image processing system (150, 250) of any one of the preceding claims, configured to receive said time series (120, 320) of images (122a, 122b, 122c, 124a, 124b, 124c, 322a, 322b, 322c, 322d) from a surgical microscope (102) or an image detector (106a, 106b) of a surgical microscope (102).

15. A controller for a surgical microscope (102), configured as or comprising the image processing system (150, 250) of any one of the preceding claims.

16. A surgical microscope system (100, 200), comprising a surgical microscope (102), an image detector (106a, 106b) and the controller of claim 15.

17. The surgical microscope system (100) of claim 16, further comprising a display (160) configured for displaying said data, which comprises information characteristic for blood flowing through a blood vessel in said tissue.

18. A method for analysing images of a tissue for blood flow, comprising:

   Receiving (508) a time series (120, 320) of images (122a, 122b, 122c, 124a, 124b, 124c, 322a, 322b, 322c, 322d) of a tissue (330);
   determining (510) a region (336) in each of said images (122a, 122b, 122c, 124a, 124b, 124c, 322a, 322b, 322c, 322d), in which a colour or intensity varies over time;
   generating (514), based on said region, data (128, 228), which comprises information characteristic for blood flowing through a blood vessel in said tissue; and
   providing (516) said data (128, 228).

19. The method of claim 18, further comprising: acquiring the time series (120, 320) of images, before receiving, by means of an image detector (106a, 106b) of a surgical microscope (102).

20. A computer program with a program code for performing the method of claim 18 or 19, when the computer program is run on one or more processors or the image processing system (150, 250) of any one of claims 1 to 14.

**Fig. 1**

**Fig. 2**

Fig. 3

Fig. 4

**Fig. 5**

# EUROPEAN SEARCH REPORT

Application Number

EP 22 15 5813

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VASSALLO REID ET AL: "Determining blood flow direction from short neurovascular surgical microscope videos", HEALTHCARE TECHNOLOGY LETTERS, vol. 6, no. 6, 31 December 2019 (2019-12-31), pages 191-196, XP006088052, DOI: 10.1049/HTL.2019.0080 * the whole document * | 1-20 | INV. A61B5/026 A61B90/20 |
| X | YU JINGYI ET AL: "Label-free Intraoperative Blood Flow Imaging and Augmented Reality Display in Surgical Microscope", 2021 10TH INTERNATIONAL IEEE/EMBS CONFERENCE ON NEURAL ENGINEERING (NER), IEEE, 4 May 2021 (2021-05-04), pages 222-225, XP033920493, DOI: 10.1109/NER49283.2021.9441393 [retrieved on 2021-05-25] * abstract * | 1-20 | |
| X | US 2010/042000 A1 (SCHUHRKE THOMAS [DE] ET AL) 18 February 2010 (2010-02-18) * paragraph [0025] - paragraph [0037] * | 1-20 | TECHNICAL FIELDS SEARCHED (IPC)<br><br>A61B |

The present search report has been drawn up for all claims

| Place of search | Date of completion of the search | Examiner |
|---|---|---|
| The Hague | 13 July 2022 | Van Dop, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 5813

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2022

| Patent document cited in search report | Publication date | Patent family member(s) | Publication date |
|---|---|---|---|
| US 2010042000 A1 | 18-02-2010 | DE 102008040807 A1 | 04-02-2010 |
|  |  | US 2010042000 A1 | 18-02-2010 |

For more details about this annex : see Official Journal of the European Patent Office, No. 12/82

EPO FORM P0459